# EUROPEAN PATENT APPLICATION

(11) **EP 2 538 197 A2**
(43) Date of publication of application: **26.12.2012**
(21) Application number: 12380034.4
(22) Date of filing: 22.06.2012
(51) Int. Cl.: G01N 3/56, G01N 19/04, G01N 33/32

(54) **Method for simulteously conducting multiple abrasion tests on products applicable on roads**

(30) Priority: 22.06.2011 ES 201131047
(71) Applicant: Sovitec Iberica, S.A.U., 08755 Castellbisbal (ES)
(72) Inventor: Calavia Redondo, David, 08755 Castellbisbal (ES); Dujardin, Stephan, 08755 Castellbisbal (ES)
(74) Representative: Espiell Volart, Eduardo Maria

(57) **Abstract**

This application discloses a method which comprises: (a) the provision of a test plate equipped with, on one of its faces, differentiated sections with different roughnesses and textures, corresponding to different types of pavements or asphalts for roads; (b) the application on the differentiated sections of the test plate of the different products to be tested, so that each one of said products covers a part of each and every one of the differentiated sections of the plate; (c) the drying or curing of the products applied on the test plate; and (d) the placement of the test plate bearing the products to be tested in a testing machine so that the face of the plate bearing the products to be tested is submitted to an abrasion test and subsequent evaluation.

## Description

### OBJECT OF THE INVENTION

The present invention relates to a method for simultaneously conducting multiple abrasion tests on products applicable on roads, consisting of comparing the durability of several products applied in different proportions and under different pavement conditions of roads.

### SCOPE OF THE INVENTION

This method is applicable in the field of the performance of abrasion tests on products applicable to roads, and more specifically for quality control and product testing for horizontal signalling and road markings on the surface of roads.

### BACKGROUND OF THE INVENTION

Many products applicable to roads such as paints, thermoplastic reliefs and others are made by means of the variable proportional mixture of different components, modifying the mechanical properties according to the requirements of the application.

Once the product is applied on the road, said product must be sufficiently resistant to withstand the wear produced by traffic and the elements during the period of time that must pass until the next maintenance operation is to be performed on the road. Furthermore, the roughness and texture of the pavement of the road affect said abrasive resistance of the applied product. Thus, the operating life of the product is difficult to determine a priori by theoretical means.

Currently, testing is known of products applicable to roads in accelerated abrasion testing machines or simulators that reproduce intense traffic conditions of vehicles on the product to be tested.

To do this, flat plates are used, on one of whose faces the product is placed, whether it is paint, a thermoplastic relief or any other surface finishing coating. These plates are inserted in the abrasion testing machine and after a series of cycles deemed suitable, the plate is removed to observe the resistance of the tested product.

The main drawback of this technique consists in that only one product can be tested on each plate. Furthermore, these plates do not make it possible to control the wear on pavements of roads with different roughnesses, which limits the validity and usefulness of the data obtained from the test.

A single session should be able to permit the simultaneous testing of different products applied in different proportions. Another drawback to resolve is that of reducing the costs of performing said tests and achieving a multiparameter set of results on the performance of different products on different types of asphalts or pavements.

These tests are very expensive and the abrasion testing machines feature a limited plate capacity, which makes it even more difficult and costly to obtain minimally coherent results from the tests for their direct and reliable comparison.

The applicant of the present invention is unaware of the existence of references which resolve the problems set forth in a satisfactory manner.

### DESCRIPTION OF THE INVENTION

The method for simultaneously conducting multiple abrasion tests on products applicable on roads features special technical features intended to provide testing means which make it possible to directly compare the performance of different products on different pavement conditions used on the roads, providing a comparative abrasion test by performing a large quantity of tests in a single session and in a more economical manner.

According to the invention, the method comprises:
a) the provision of a test plate equipped with, on one of its faces, differentiated sections with different roughnesses and textures, corresponding to different types of pavements or asphalts for roads;
b) the application on the differentiated sections of the test plate of the different products to be tested, so that each one of said products covers a part of each and every one of the differentiated sections of the plate;
c) the drying or curing of the products applied on the test plate; and
d) the placement of the test plate bearing the products to be tested in a testing machine so that the face of the plate bearing the products to be tested is submitted to the abrasion test.

In this way, several products that are to be compared are placed on a single plate and simultaneously submitted to the same abrasion test. Therefore, it can be declared that comparing the results obtained from the test will definitely lead to one choosing the most suitable product. This reduces the number of test plates to be used.

Additionally, as each plate features several sections with different roughnesses and textures simulating different pavements on its face whereto the products are applied, the test allows one to determine the performance of the different combinations of products and pavements. Therefore, the method of the invention simplifies the performance of the tests and facilitates the comparison and evaluation of the results obtained.

### DESCRIPTION OF THE FIGURES

To complement the description that is being made and with the object of assisting in a better understanding of the characteristics of the invention, accompanying the present specification is a set of drawings wherein, by way of illustration and not restrictively, the following has been represented:

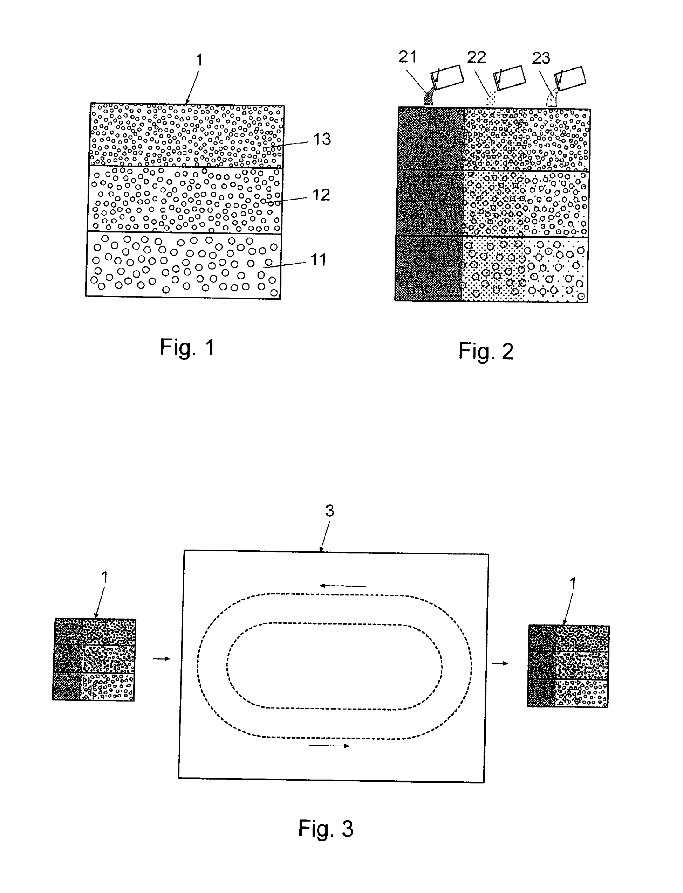

- Figs. 1, 2 and 3: schematically show the consecutive stages of the method for simultaneously conducting multiple abrasion tests on products applicable on roads, according to the invention.

### PREFERRED EMBODIMENT OF THE INVENTION

In the first place, the method comprises the provision of a test plate (1) equipped with, on one of its faces, differentiated sections (11, 12, 13) with different roughnesses and textures, corresponding to different types of pavements or asphalts for roads. In the example shown, this plate (1) is obtained by molding, featuring three differentiated sections (11, 12, 13) with reliefs or roughnesses of greater or lesser calibre.

Then, as shown in figure 2, the different products to be tested are applied on the differentiated sections (11, 12, 13) of the plate (1), referenced in this case as (21, 22, 23), so that each one of said products (21, 22, 23) covers a part of each and every one of the differentiated sections (11, 12, 13) of the plate (1), then waiting for the drying or curing of the products applied on the test plate to be produced.

Finally, the test plate (1) bearing the products to be tested (21, 22, 23) are placed in an abrasion testing machine (3) so that the plate bearing the products to be tested is submitted to an abrasion test.

Once the plate (1) has been submitted to the abrasion test, a multiparameter set of results (1') is obtained, as one can observe the performance of each and every one of the products (21, 22, 23) on the different types of pavements reproduced on the sections (11, 12, 13) of the test plate (1). This permits the most suitable product (21, 22 or 23) to be chosen for application on a specific road, firstly determining which is the differentiated section (11, 12 or 13) which features the most similar characteristics to the road in question and then observing which product (21, 22 or 23) has produced the best result in that specific differentiated section of the plate.

The nature of the invention having been sufficiently described, as well as a preferred example of embodiment thereof, it should be mentioned for the appropriate purposes that the materials, shape, size and arrangement of the elements described can be modified, as long as it does not result in an alteration of the essential characteristics of the invention, which are claimed below.

## Claims

1. Method for simultaneously conducting multiple abrasion tests on products applicable on roads, **characterized in that** it comprises:
a) the provision of a test plate (1) equipped with, on one of its faces, differentiated sections (11, 12, 13) with different roughnesses and textures, corresponding to different types of pavements or asphalts for roads,
b) the application on the differentiated sections (11, 12, 13) of the test plate of the different products (21, 22, 23) to be tested, so that each one of said products (21, 22, 23) covers a part of each and every one of the differentiated sections (11, 12, 13) of the plate,
c) the drying or curing of the products (21, 22, 23) applied on the test plate (1), and
d) the placement of the test plate (1) bearing the products (21, 22, 23) to be tested in a testing machine (3) so that the face of the plate (1) bearing the products (21, 22, 23) to be tested is submitted to an abrasion test and a subsequent evaluation.
